# EUROPEAN PATENT SPECIFICATION

(11) **EP 2 986 141 B1**
(45) Date of publication and mention of the grant of the patent: **03.07.2019**
(21) Application number: 14724328.1
(22) Date of filing: 17.04.2014
(51) Int. Cl.: A23L 5/00, A23L 33/10, A61K 9/107, A61K 9/16, A23P 10/30, A23L 33/115

(54) **ENCAPSULATION OF AN OIL CONTAINING UNSATURATED FATTY ACIDS**
VERKAPSELUNG EINES ÖLS MIT UNGESÄTTIGTEN FETTSÄUREN
ENCAPSULATION D'UNE HUILE CONTENANT DES ACIDES GRAS INSATURÉS

(30) Priority: 19.04.2013 EP 13164581
(43) Date of publication of application: 24.02.2016
(73) Proprietor: MEINERS,, Jean Antoine, 2036 Cormondrèche (CH)
(72) Inventor: ZONGO, Mathieu, CH-1018 Lausanne (CH); MEINERS, Jean Antoine, CH-2036 Cormondrèche (CH); ANGLADA, Angela, CH-2000 Neuchâtel (CH)
(74) Representative: Schneiter, Sorin
(86) International application number: PCT/EP2014/057976
(87) International publication number: WO 2014/170464

(56) References cited:
- US-A1- 2004 017 017
- US-A1- 2007 141 211
- US-B1- 6 444 242
- RIITTA PARTANEN: "Mobility and oxidative stability in plasticized food matrices. The role of water", VTT PUBLICATIONS, no. 697, 2008, pages 1-92, XP002716508, Helsinki, Finland ISSN: 1235-0621 ISBN: 978-951-38-7125-3

## Description

### Technical Field

The present invention relates to the stabilization of a material comprising unsaturated fatty acids, in particular polyunsaturated fatty acids (PUFAs), such as omega 3 and/or omega 6 fatty acids, in particular those from marine oil origin. The present invention relates also to ways for masking undesired odours and to ways for improving the taste of materials containing unsaturated fatty acids. More specifically, the invention relates to microcapsules comprising unsaturated fatty acids, to a method for preparing the microcapsules and a composition, for example a food product, comprising the microcapsules.

### Prior Art and the Problem Underlying the Invention

It is common knowledge that unsaturated fatty acids, in particular PUFAs, lack stability, since the double bonds are easily disrupted. The hydrogen atom at the methylene group can be removed to form an alkyl radical, which may start a chain reaction. A certain stabilization of materials comprising unsaturated fatty acids is obtained by adding antioxidants and free radical scavengers.

Consequently, the scientific community has been searching for a few decades for more powerful methods for combatting oxidation of PUFA oils. Microencapsulation is a technology that shields the core active ingredient from its environment by creating a seamless wall, the shell or the matrix. Of course, microencapsulation suitable for consumption must use shell and matrix materials that are non-toxic and approved for its application purpose as a pharmaceutical, food or feed excipient, as the case may be. Preferably, the materials or ingredients of the microcapsules should be readily available at reasonable cost. Ideally, the process to apply the shell or matrix material around the material comprising the unsaturated fatty acids should be cost efficient.

Spray-drying is a technology that can be used for microencapsulation. This method has the merit of high production capacities and reasonable production costs. On the other hand, process temperatures are generally moderate to high, which may be a problem with respect to the encapsulation of unsaturated fatty acids.

GB 2031937 discloses a process of spray-drying of an emulsion comprising liquid fats emulsified in a caseinate matrix. A process for spray drying marine oil in a matrix of caseinate and starch is disclosed in EP 0425213. DE 69306576 T2 discloses the spray-drying of an oil with a high content of unsaturated fatty acids in a matrix of caseinate and a carbohydrate. In WO 2008/066380 a process for encapsulating oil-in-water emulsions using denatured proteins is disclosed, wherein said oil contains unsaturated fatty acids.

Kolanski et al., Sciences and Nutrition 2004, vol. 55, no. 4, pages 333-343 produced oil microcapsules by spray-drying of homogenized emulsions containing modified celluloses and maltodextrin as coating materials. Methylocellulose (MC) and hydroxypropyl methylocellulose (HPMC) showed good emulsifying properties. Homogenisation of emulsions resulted in creation of high amount of foam. More damage occurred in the powders coated with HPMC. Samples with fish oil content of approximately 500 g/kg exhibited more structural damage, which impaired the stability of the fish oil. US2004/017017 discloses a method for encapsulating fish oil comprising the steps of forming a micro emulsion comprising water, a polymer (e.g. starch), the hydrophobic material, an emulsifier and a plasticizer. The oil droplets size in the emulsion is in the range of from 0.5 to preferably less than 2 micrometers. The emulsion is then spray dried and optionally further coated by fluidized bed.

It is an objective to provide a food product that is enriched in unsaturated fatty acids whereby, upon consumption, the consumer does not become aware of the additive comprising the unsaturated fatty acids. In particular, the consumer preferably does not become aware of any difference in taste due to an off-taste of the oil, nor should there be any noticeable textural difference or particularity due to the presence of a unsaturated fatty acids supplement or of capsules containing unsaturated fatty acids. It is a goal of the invention to provide capsules that are not notices upon consumption. Capsules are generally noticeable upon consumption by a consumer when they have a size that is larger than 20 µm, sometimes only at about 50 µm, depending on the hardness of the capsules. The factors playing a role with respect to perception are thus capsule size and hardness. It is an objective of the invention to provide softer capsules.

Preferably, a powder comprising capsules should not be perceived in the mouth as "sandy" and therefore have a small particle size.

It is an objective to provide unsaturated fatty acids in a stable, preferably dry form allowing its addition to products destined to oral consumption. It is a further objective to provide unsaturated fatty acids in a form in which the presence of undesired off-tastes are reduced.

It is a further objective of the invention to provide a process of encapsulation of unsaturated fatty acids, resulting in a more stable ingredient, preferably exhibiting less off-taste.

The present invention addresses the problems depicted above.

Krill oil has been rejected in many health promoting formulations, for reason of its objectionable strong shrimp like flavor and odor. Even at low inclusion rates such objectionable olfactory characteristic are too strong to be masked with other ingredients. It is thus an objective of the invention to provide Krill oil in a manner that its undesired odors or flavors are masked or not apparent. It is an objective to provide a manner of encapsulating Krill oil, allowing its use as an additive in food, nutraceutical or pharmaceutical products.

### Summary of the Invention

Remarkably, the inventors address the problems above by capsules and a process for preparing the same. Surprisingly, the unsaturated fatty acids in the capsules according to the invention are more stable, and the capsules exhibit less oxidation and less off-taste. Interestingly, the capsules can be obtained by a spray-drying process.

Surprisingly, the present invention fulfills the criteria with respect to perception and oral sensation without the commonly known presence of surface oil on the outer side of the microcapsules.

Surprisingly, the taste and odor of krill oil, encapsulated according to the present invention was pre-dominantly masked over a time period of at least one year.

In an aspect, the invention provides a process for producing capsules, the process comprising the steps of: 1) preparing an emulsion comprising water, one or more polymers, a hydrophobic material, and at least one emulsifier; and, 2) spray-drying the emulsion.

In an aspect, the invention provides a process for producing capsules, the process comprising the steps of: 1) preparing an emulsion comprising water, one or more polymers, a hydrophobic material comprising an oil comprising unsaturated fatty acids, such as a marine oil and/or a modified marine oil, and at least one emulsifier; and, 2) spray-drying the micro emulsion.

In an aspect, the invention provides a process for microencapsulating a hydrophobic material comprising PUFAs, the method comprising the steps of: preparing a micro emulsion comprising water, one or more polymers, said hydrophobic material, and at least one emulsifier, wherein said hydrophobic material forms droplets having an average size of 1 µm or smaller in said micro emulsion; adding at least one liposoluble plasticizer, said plasticizer providing at least 2% per weight of said micro emulsion, determined with respect to the weight of non-aqueous constituents of the micro emulsion containing all constituents and/or immediately before spray drying; and, spray-drying said micro emulsion.

In an aspect, the invention provides microcapsules obtainable by any one of the processes of the invention.

In an aspect, the invention provides a microcapsule comprising a matrix comprising polymers and droplets of a hydrophobic material dispersed in said matrix, said droplets having an average diameter of 1 µm or smaller, said hydrophobic material comprising PUFAs, wherein said hydrophobic material provides at least 10% per weight of dry matter of said microcapsule, characterized in that said microcapsule comprises a plasticizer, which provides at least 2 % per weight of dry matter of said microcapsule.

In an aspect, the invention provides an ingestible product for example a food product, comprising the microcapsules of the invention.

### Detailed Description of the Preferred Embodiments

In some of its aspects, the present invention provides methods of and processes for encapsulation, capsules and compositions comprising the capsules. Preferably, said capsules are microcapsules and said process is a process for microencapsulation. The processes of the invention are preferably processes of encapsulation, in particular microencapsulation, and even nano-encapsulation, of a hydrophobic material.

The invention provides a process for producing capsules, the process comprising the steps of: 1) preparing an emulsion, preferably a basic emulsion, comprising water, one or more polymers, a hydrophobic material, and at least one emulsifier; and, 2) spray-drying the emulsion.

For the purpose of the present specification, the expression "comprising" and various grammatical forms thereof is intended to mean "includes, amongst other". It is not intended to mean "consists only of".

All constituents, ingredients and/or components other than added water that are added to the mixture and/or emulsion before the step of spray-drying the emulsion form the total of the non-aqueous constituents of said emulsion. Examples of said non-aqueous constituents of said emulsion are polymers, the hydrophobic material, emulsifiers, plasticizers, and polyalcohols.

The emulsion can be prepared as is conventional, by mixing non-aqueous constituents or ingredients with water and emulsifying the obtained mixture in a second step. The non-aqueous constituents may be added to a recipient and mixed with the water. As specified elsewhere in this specification, some (preferably non-aqueous) constituents of the capsules may also be added after a basic emulsion is formed. These constituents still are constituents of the emulsion before spray-drying. This may apply in particular to constituents that are not required for the formation of the emulsion as such. Polymers, or at least a part of thereof, the hydrophobic material, the emulsifier and water are generally beneficial for forming the emulsion and are thus preferably mixed before emulsification.

In an embodiment of the invention, the step of preparing the emulsion to be spray-dried comprises a step of mixing water and a first part of non-aqueous constituents of the emulsion, and emulsifying the mixture obtained in the previous step so as to provide a basic emulsion. In a subsequent step, second or further parts of said non-aqueous constituents, and optionally further water, for example aqueous solutions, may be added to the basic emulsion so as to provide a final emulsion to be spray-dried. For example, non-aqueous constituents and water may be added to the basic emulsion in the form of an aqueous solution of the non-aqueous constituent, so as to provide said final emulsion.

As will be discussed with respect to some of the embodiments of the invention, at least one of the plasticizers may be added to the basic emulsion, that is, once a basic emulsion is prepared, for example.

The constituents for the emulsion are preferably selected from ingredients and/or excipients such that the natural biochemistry in the digestive tract will allow release and/or bioavailability of the active ingredients, in particular of the PUFAs. Furthermore, the quantity of the ingredients and/or excipients of the emulsion and/or capsules are selected such release and bioavailability of the active ingredients is warranted.

The invention relates to capsules containing a hydrophobic material and to a process for encapsulating a hydrophobic material. Preferably, the hydrophobic material comprises unsaturated fatty acids (UFAs), in particular polyunsaturated fatty acids (PUFAs). PUFAs encompass omega-3 and omega-6 fatty acids, for example. Preferably, the hydrophobic material comprises one or more selected from eicosapentaenoic acid (EPA), docosahexaenoic acid (DHA), arachidonic acid (ARA), linoleic (or lipoic) acid (LA), and linolenic acid, or mixtures comprising two or more of the aforementioned. Preferably, the hydrophobic material is an oil comprising UFAs, in particular PUFAs.

Preferably, the hydrophobic material comprises at least 3% by weight, preferably at least 5% by weight, more preferably at least 10% by weight and even more preferably at least 15% by weight of UFAs, in particular PUFAs. Most preferably, the hydrophobic material comprises 15% by weight, 20% by weight or even 30% by weight or more of UFAs, in particular PUFAs.

Oils containing PUFAs are commercially available. According to a preferred embodiment, said hydrophobic material comprises marine oil, preferably krill oil and/or fish oil and/or modified marine oil. Some of these oils, in particular Krill oil, contain phospholipids.

Oils containing PUFAs can also be obtained from plants. PUFAs can thus be provided in the form of plant oils.

Hydrophobic materials containing unsaturated fatty acids, in particular marine oils, are prone to stability problems due to undesired oxidation, in particular at elevated temperatures as they generally occur during spray-drying. Therefore, the hydrophobic material is preferably supplemented with additives aiming at increasing the stability of the hydrophobic material, in particular during processing in accordance with the invention, for example spray-drying, but also during storage.

According to an embodiment, the hydrophobic material comprises antioxidants. Preferably, said antioxidants of said hydrophobic material are hydrophobic and/or liposoluble antioxidants.

Preferably, the hydrophobic material comprises one or more primary antioxidants (such as a free radical scavenger (interceptors, reducing agents)) and one or more secondary antioxidant, the latter being preferably selected from oxygen scavengers, metal chelators and singlet oxygen quenching agents. In particular, the hydrophobic material comprises, one or more free radical scavenger, and, in addition, one or more selected from oxygen scavengers, metal chelators singlet oxygen quenching agents, and combinations thereof.

Examples of free radical scavengers are tocopherols, tocotrienols, and plant extracts, in particular extracts of plants of the mint family (Lamiaceae). Typically, rosemary extract is used.

An example of a (liposoluble) oxygen scavenger is ascorbyl palmitate. Examples of liposoluble singlet oxygen quenching agents are carotenoids (beta-carotene, lycopene and lutein). Examples of metal chelators are lecithin and citric acid.

According to an embodiment, the hydrophobic materials comprises one, two or more different primary antioxidants and, in addition, at least one or at least two selected from the group of oxygen scavengers, metal chelators and singlet oxygen quenching agents, preferably at least one oxygen scavenger and at least one metal chelators.

While the hydrophobic material preferably comprises hydrophobic antioxidants of the above mentioned types, the invention also encompasses the use of hydrophilic antioxidants, for example in the aqueous phase of the emulsion. Examples of hydrophilic metal chelating agents are phytic, malic, succinic and tartaric acids and EDTA. Examples of hydrophilic oxygen scavengers are ascorbic acid, erythorbic acid, sodium erythorbate and sulfites.

According to an embodiment, additives contained in the hydrophobic material are food grade and/or preferably pharma-grade. More specifically, all constituents of the microcapsules of the invention are preferably food grade and/or pharma grade.

According to an embodiment of the process of the invention, said hydrophobic material provides from 5% to 50% by weight of the non-aqueous constituents of the emulsion. Preferably, the emulsion comprises, in percent by weight of the total of all non-aqueous constituents before spray drying, 10% to 50%, preferably 15% to 45%, more preferably 20% to 40% and most preferably 25% to 35% of hydrophobic material, in particular oil comprising unsaturated fatty acids.

The total (100%) of the non-aqueous constituents of the (final) emulsion before spray-drying in the process of the invention will substantially constitute the dry-matter of the microcapsules of the invention. Therefore, the percentage of a non-aqueous constituent in the emulsion (for example: 25% hydrophobic material) also corresponds to the percentage of the respective constituent with respect to the total dry-matter of the capsules of the invention.

The weight of non-aqueous constituents is preferably the weight of the constituent as commercially obtained. Such non-aqueous constituents may comprise some residual water, which is part of the weight. According to an embodiment, the weight of a non-aqueous constituent is weight of the dry-matter only of said non-aqueous constituent. The dry-matter weight may be determined by subtracting residual water present in the non-aqueous constituent or by drying the constituent until all water is removed.

The expression "before spray-drying", for example in the context of the total of the non-aqueous constituents of the emulsion "before spray-drying", refers to the moment when the water and all non-aqueous constituents have been added to provide the final emulsion, immediately before spray-drying. In other words, "the total of the non-aqueous constituents of the emulsion before spray-drying" refers to the total of the non-aqueous constituents that are present in the final emulsion that is subjected to spray-drying in a subsequent step.

Percentages with respect to the emulsion given in this specification generally refer to percentages by weight of all and/or the total of the non-aqueous constituents of the final emulsion before spray-drying.

In an embodiment, the microcapsules of the invention comprise, in percent by weight of total dry matter, from 5% to 50%, preferably 10% to 50%, more preferably 15% to 45%, even more preferably 20% to 40% and most preferably 25% to 35% of hydrophobic material, in particular oil comprising unsaturated fatty acids.

It is surprising that stable microcapsules can be obtained with as high loads of about 25% to 45% by weight of marine oil.

The capsules of the invention comprise polymers, and the process of the invention comprises the step of preparing an emulsion comprising at least water, one or more polymers, a hydrophobic material, and at least one emulsifier.

The state of the art proposes various polymers suitable as shell- or matrix material of microcapsules and it would go beyond the scope of the present specification to enlist all polymers that can be used for the purpose of the present invention. Preferably, at least one of the polymers present in the capsules or used in the process of the invention is selected from the groups consisting of polysaccharides, modified polysaccharides, polypeptides and modified polypeptides. In other words, the polymer preferably comprises, consists essentially of or consists of monosaccharide moieties and/or amino acid moieties, whereby said moieties may or may not be modified, for example derivatives. For example, said moieties may be derivatized by attachment of one or more chemical groups that modify the properties of the polymer.

According to an embodiment, the polymers comprise proteins, in addition and/or instead of said polysaccharides and/or modified polysaccharides. Said proteins may also be modified.

It is preferred that the emulsion prepared in the process of the invention is a stable emulsion, in which the hydrophobic material forms droplets of a defined size, as specified elsewhere in this specification. Therefore, it is preferred to use polymers having emulsifying properties.

According to an embodiment, said polymers provide from 30% to 85% by weight of the non-aqueous constituents of the emulsion. According to an embodiment of the process, the emulsion preferably comprises, in percent by weight of the total of all non-aqueous constituents before spray drying, 30% to 80%, preferably 35% to 70%, more preferably 40% to 65% and most preferably 45% to 60% of polymers, preferably polysaccharide or modified polysaccharide polymers. These percentages also refer to the dry matter content of polymers in the capsules of the invention.

According to an embodiment, the polymers comprise at least one polymer selected from cellulose, cellulose derivatives and pullullan. According to a preferred embodiment, the polymers comprise at least one polymer selected from cellulose derivatives and pullullan.

According to an embodiment, said cellulose derivatives are preferably selected from alkylated, hydroxyalkylated and/or carboxyalkylated cellulose. According to a preferred embodiment, said cellulose derivative is selected from the group of methyl cellulose, ethylcellulose, hydroxypropylcellulose (HPC), hydroxypropyl methyl cellulose (HPMC), hydroxyethylmethylcellulose (HEMC), carboxymethylcellulose and mixtures comprising two or more of the aforementioned.

According to an embodiment, the polymers comprise at least one polymer selected from cellulose derivatives, in particular from those specified in this specification.

In the process of the present invention, the emulsion preferably comprises, in percent by weight of the total of all non-aqueous constituents before spray drying, from 5% to 70%, preferably 10% to 60%, more preferably 15% to 50%, even more preferably 17% to 40% and most preferably 20% to 35% of one or more selected from cellulose, cellulose derivatives and pullulan, preferably cellulose derivatives, such as one or more of the cellulose derivatives specified in this specification. According to a most preferred embodiment, the above percentages apply to the presence of HPMC in the emulsion before spray-drying.

In an embodiment, the microcapsules of the invention comprise, in percent by weight of total dry matter, from 5% to 70%, preferably 10% to 60%, more preferably 15% to 50%, even more preferably 17% to 40% and most preferably 20% to 35% of one or more selected from cellulose, cellulose derivatives and pullulan, preferably cellulose derivatives, such as one or more of the cellulose derivatives specified in this specification. According to a most preferred embodiment, the above percentages apply to the presence of HPMC in the microcapsules.

According to an embodiment, the polymers comprise at least one polymer selected from natural gums. According to an embodiment, the natural gum is selected from the group of gum arabic, guar gum, gum tragacanth, gum ghatti, karaya gum, mastic gum, gellan gum, xanthan gum and mixtures comprising two or more of the aforementioned. Preferably, the natural gum comprises gum arabic.

In an embodiment of the process of the present invention, the emulsion preferably comprises, in percent by weight of the total of all non-aqueous constituents before spray drying, from 5% to 70%, preferably 10% to 60%, more preferably 15% to 50%, even more preferably 17% to 40% and most preferably 20% to 35% of natural gums, preferably gum arabic.

In an embodiment, the microcapsules of the invention preferably comprise, in percent by weight of the total dry matter of the microcapsules, from 5% to 70%, preferably 10% to 60%, more preferably 15% to 50%, even more preferably 17% to 40% and most preferably 20% to 35% of natural gums, preferably gum arabic.

According to a preferred embodiment, the polymer comprises a combination of:
(A) one or more selected from cellulose derivatives and pullulan; and,
(B) a natural gum.

Preferably, the polymer comprises (A) a cellulose derivative as specified elsewhere in this specification, preferably HPMC, and (B) a natural gum, preferably gum arabic.

In the emulsion and/or the microcapsules, said polymers (A) and (B) are preferably present at a weight ratio r = ((A)/(B)), wherein r is a value within the range of 0.25-4, preferably ⅓-3, more preferably 0.5-2, even more preferably 0.75- 1⅓. More preferably, said r is in the range of about 0.8-1.2, preferably 0.9-1.1. For example, (A) and (B) are preferably present at about an identical quantity (r ≈ 1) in the emulsion and/or in the microcapsules of the invention.

In the process of the invention, the emulsion preferably comprises, in percent by weight of the total of the non-aqueous constituents of the emulsion before spray-drying, at least 10% by weight of said cellulose derivate and/or pullulan, and, in addition, at least 10% by weight of said natural gum. These weight percentages also apply to the dry-matter content of cellulose derivate and/or pullulan and natural gum in the microcapsules of the invention.

In the process of the invention, the emulsion preferably comprises, in percent by weight of the total non-aqueous constituents of the emulsion before spray-drying, 10% to 40%, preferably 15% to 35%, more preferably 20% to 30% and most preferably 22% to 28% of (A) and 10% to 40%, preferably 15% to 35%, more preferably 20% to 30% and most preferably 22% to 28% of (B). These weight percentages also apply to the dry-matter content of (A) and (B) in the microcapsules of the invention.

In another embodiment of the process of the invention, the emulsion preferably comprises, in percent by weight of the total of the non-aqueous constituents of the emulsion before spray-drying, 15 to 30% by weight of dry matter of said cellulose derivate and/or pullulan and, in addition, 15 to 30% by weight of dry matter of said natural gum. These weight percentages also apply to the dry-matter content of cellulose derivate and/or pullulan and natural gum in the microcapsules of the invention.

Preferably, the process of the invention comprises the step of preparing an emulsion, in particular an oil-in-water emulsion (O/W). Preferably, the emulsion is formed so that said hydrophobic material forms droplets having an average size of 1 µm or smaller in said emulsion. Said emulsion is a micro-emulsion. In order to achieve the desired droplet size, an emulsifier or, preferably, an emulsifier system is used. The skilled person is able to choose emulsifiers suitable to adjust the droplet size of the hydrophobic material in accordance with the invention. Examples of emulsifiers that may be used at a specific quantity to adjust the droplet size in accordance with the invention are Tween® 20 (polysorbate 20), Span® 85 (sorbitan trioleate), and/or sucrose esters, such as Sisterna PS750® and Sisterna SP50®. As the skilled person will understand, the emulsifier system is preferably selected in dependence of other constituents of the emulsion, in particular the polymers and, if applicable, the plasticizers.

As the skilled person will understand, the choice and/or selection of the emulsifiers and/or the emulsifier system is the result of the optimization with respect to the required HLB of the hydrophobic material. Generally, an emulsifier with a comparatively high and another emulsifier having a comparatively low HLB value are selected. The skilled person will appreciate that the emulsifying and/or co-emulsifying properties of the constituents of the emulsion, such as the polymers, and, in as far as present, the plasticizers, co-plasticizers and polyhydric alcohol are preferably measured and evaluated by appropriate surface tension analysis, such as spinning drop assay, for example.

According to an embodiment, the emulsion comprises at least two emulsifiers, one of which having an HLB (Hydrophilic Lipophilic-Balance) value of >10 and the other having an HLB value <10. According to an embodiment, the emulsion comprises a first emulsifier and a second or co-emulsifier.

According to an embodiment, the emulsion comprises, in percent by weight of the total of the non-aqueous constituents of the emulsion before spray-drying, 2 to 15%, preferably 3 to 10% of the weight of said emulsifiers, including said co-emulsifier, if present. These percentages also refer to the dry matter content of emulsifiers in the capsules of the invention.

According to a preferred embodiment, said hydrophobic material forms droplets having a mean diameter of 1 µm or smaller, preferably 750 nm or smaller, more preferably 500 nm or smaller in said emulsion. For example, the mean droplet diameter is from 50 nm to 1 µm. Most preferably, the mean droplet diameter is 400 nm or smaller or even 300 nm or smaller. According to an embodiment, the mean droplet diameter is in the range of 100 to 750 nm. The process of the invention preferably comprises the step of producing an emulsion having the droplet size as specified and/or the step of adjusting the droplet size in the emulsion to the droplet size specified in this specification.

The emulsion prepared in the process of the invention is preferably stable. "Stable" for the purpose of the emulsion, means that the average droplet size in the emulsion does not change by more than 30% following storage of the emulsion at room temperature (25°C) for 3 days, preferably 1 week, most preferably 2 weeks.

According to an embodiment, said emulsion is stable so that when stored at room temperature (25°C) for one week, a mean diameter of said droplet size does not change by more than 10%. The terms "mean" or "average" preferably refer to the arithmetic mean or average.

The droplet size (the mean droplet diameter) of an emulsion may be determined by Dynamic Light Scattering analysis (DLS) as specified in the examples hereinafter. The desired stability can be achieved by adjusting the droplet size and/or selecting an emulsifier system as indicated elsewhere in this specification.

The droplet size can also be analyzed in the microcapsules of the invention. Accordingly, the microcapsules are broken and analyzed by electron-microscopy. The average droplet size can be determined by measuring the diameter of a representative sample of droplets on photographs made using electron-microscopy.

The present inventors found that better results were obtained when the emulsion that was later subjected to spray drying was stable before spray-drying. This is surprising, because generally the stability of the emulsion is not known or reported to have any impact on the stability of the capsules obtained by or following spray-drying. Optimal stability can be achieved by forming mono dispersed droplet size. In practical terms droplets over 5 µ, potentially over 1 µ will coalescence over a relative short period of time (hours). Therefore a mono dispersed droplet size of < 1 µ, preferably < 500 nm are preferred.

The process of the present invention comprises the step of spray-drying the emulsion prepared in accordance with a preceding step. Spray-drying may be done as is conventional. In general, the emulsion may be pushed through a nozzle resulting in the atomization and/or suspension of the emulsion in the form of small droplets/aerosols. The emulsion may be sprayed (or atomized, dispersed) into a recipient called the spray-drying tower. The emulsion may preferably be sprayed into hot air, so that the aqueous phase of the emulsion is dried and assumes a glassy state.

In accordance with the invention, co-current or counter current air streams may be used, for example, with atomizing devices of different configuration, such as two-fluid nozzles, rotary nozzles or ultrasonic nozzles, such as known by those skilled in the art.

The particles and/or capsules of the invention preferably have a mean diameter of from 0.5 to 100 µm, preferably from 1 to 60 µm, more preferably from 2 to 50 µm, even more preferably from 2.5 to 30 µm. According to an embodiment, the particles and/or capsules of the invention have a mean diameter of from 1 to 20 µm. The mean diameter of the particles is preferably determined in accordance with ISO 13320-1 using the NICOMP TM 380 ZLS analyzer.

The process comprises the step of adding at least one liposoluble plasticizer. It has surprisingly been discovered that the inclusion of plasticizers and, optionally, one or more co-plasticizers or further additives such as polyols (polyalcohols), allow the inclusion of remarkably higher loads of hydrophobic material than reported in prior art without causing instability.

Preferably, the at least one liposoluble plasticizer is mixed with the remaining constituents forming the emulsion to be spray-dried. It was surprisingly found that by adding a plasticizer, for example to the emulsion before spray-drying, the stability of the capsules was increased, and the oxidation of the unsaturated fatty acids and the occurrence of undesired off-tastes reduced. Without wishing to be bound by theory, one potential explanation is that the plasticizer reduces the brittleness of the (micro)capsules and preserves the integrity of the droplets of hydrophobic material in the matrix of the capsules, thereby reducing leakage and/or exposure to air of the hydrophobic material.

The at least one plasticizer provides at least 2 % by weight of the non-aqueous constituents of the emulsion before spray-drying. The at least one liposoluble plasticizer is preferably added so that the emulsion preferably comprises, in percent by weight of the total of all non-aqueous constituents before spray drying, at least 2%, more preferably at least 3% and most preferably at least 4% by weight of said at least one plasticizer. The emulsion preferably comprises up to 35% by weight of plasticizers, for example up to 25% by weight.

According to an embodiment, the emulsion preferably comprises, in percent by weight of the total of all non-aqueous constituents before spray drying, from 2 % to 35%, preferably 2% to 30%, more preferably 3% to 25%, even more preferably 4% to 20%, most preferably 5% to 20% of said at least one plasticizers.

According to an embodiment of the process of the invention, said at least one plasticizer, including said co-plasticizer, if present, is added so as to provide 4% to 35%, preferably 5% to 30% by weight of all non-aqueous constituents of the micro-emulsion before spray drying. In other embodiments, the emulsion preferably comprises, in percent by weight of the total of all non-aqueous constituents before spray drying, from 6% to 35%, preferably 7% to 30%, more preferably 9% to 25%, even more preferably 11% to 20%, most preferably 11% to 20% of plasticizers.

Plasticizers are a diverse group of chemical compounds and/or compound structures that plasticize materials, that is, increase the plasticity and/or the fluidity of a material.

Essentially, plasticizer functionality is the result of modifying the molecular mobility, at the same time filling the intermolecular voids. Plasticizers generally reduce the glass transition temperature (Tg) of polymers or of a composition comprising polymers, such as the emulsion and/or the microcapsules of the invention. In addition, plasticizers generally render the material more flexible and/or less brittle.

For the purpose of an embodiment of the invention, a plasticizer is preferably a constituent that reduces the Tg of spray-dried microcapsules, compared with microcapsules lacking the plasticizers. Tg is determined by Differential Scanning Calorimetry (DSC) in accordance with ISO norm: 11357-2:1999 (E) (referring in part to ISO norm: 11357-1:1997). To determine whether a potential or candidate plasticizer constituent of the microcapsules is indeed a plasticizer in accordance with the invention, microcapsules lacking the potential plasticizer are prepared in the same manner as microcapsules containing the potential plasticizer, whereby the amount of hydrophobic material and emulsifiers is kept identical and the amount of the other constituents (polymers, if present: polyols, and so forth) is increased proportionally to compensate for the quantity of omitted potential plasticizer. The analysis is made on a sample of 10-20 mg using a temperature gradient (DSC). If the microcapsules lacking the potential plasticizer have a Tg that is significantly increased (for example at least 5°C, possibly up to 10°C or more), the potential plasticizer is a plasticizer in the sense of this invention.

A more detailed understanding of plasticizers is given in the "Handbook of Plasticizers" of George Wypych, William Andrew publishing and ChemTec Publishing defines and classifies plasticizers, 2004. In this book, plasticizers are defined and classified, mechanisms of action are given and specific plasticizers are discussed. A detailed discussion of plasticizer function would go beyond this specification.

It is noted that the choice of a particular plasticizer depends on the polymers that are used. A compound may function as a plasticizer for some polymers but not for others. In this respect, the "Handbook of Plasticizers" cited above refers to "Theories of Compatibility" (Chapter 6) finding that "Compatibility is the ability of a plasticizer to form a homogenous system with the polymer. [...] The maximum amount of a plasticizer incorporated into a polymer and retained by it without exudation is popularly accepted as the limit of compatibility". In other words, the plasticizer is preferably selected to be compatible with the polymers used for preparing the emulsion and/or the polymers forming, besides other compounds and additives, the matrix of the capsules.

For the purpose of the present specification, "water" is not considered as a plasticizer or is excluded from the definition of plasticizers, because water is a required constituent of the emulsion to be spray-dried anyhow, and residual water may also be present in the microcapsules of the invention.

In an embodiment, the plasticizer of the invention is not water nor an aqueous (water-based) liquid, such as sorbitol, fruit juice and other aqueous liquids which enable the formation of a dough.

In as far as water can be considered as being a "plasticizer", the invention comprises at least one or more plasticizers in addition to water, preferably at the quantities specified elsewhere in this specification.

The at least one plasticizer is selected from the group of liposoluble plasticizers, in particular from the liposoluble plasticizers specified elsewhere in this specification.

According to a preferred embodiment of the process of the invention, said plasticizer is added after preparing said emulsion and/or is added to the emulsion before spray-drying. Alternatively, the emulsifier can be added before or while preparing the emulsion.

According to an embodiment of the invention, said plasticizer is a principle plasticizer, and the process comprises the step of adding a co-plasticizer so as to produce an emulsion comprising at least two different plasticizers. The principle plasticizer may also be referred to as a first plasticizer and the co-plasticizer as a second plasticizer.

Said plasticizer and/or said principle plasticizer is a liposoluble plasticizer. According to an embodiment, said co-plasticizer is a water soluble plasticizer. While the invention encompasses the presence of only one plasticizer, the presence of at least two plasticizers is preferred. If only one plasticizer is used, the plasticizer is liposoluble.

The invention also encompasses that there are two or more plasticizers that are liposoluble and/or that there are two or more plasticizers that are water soluble. However, in accordance with an embodiment where there at least two plasticizers, at least one plasticizer is liposoluble and at least one other plasticizer is preferably water soluble.

For the purpose of this specification, a "water soluble plasticizer" is a plasticizer that is better soluble in water than in a hydrophobic liquid. A "liposoluble plasticizer" is a plasticizer that exhibits an increased solubility in a hydrophobic liquid.

According to an embodiment of the microcapsules of the invention, said plasticizer is a principle plasticizer and the microcapsules comprise a further plasticizer, also referred to as a co-plasticizer. Accordingly, the microcapsules of the invention comprise at least two plasticizers.

In an embodiment of the process of the invention, said step of preparing said emulsion comprises the steps of: 1) mixing said one or more polymers, water, said hydrophobic material and said at least one emulsifier to produce a mixture; and, 2) emulsifying said mixture so as to obtain said emulsion, which is preferably a basic emulsion. Preferably, said emulsifier is non-polymeric.

According to an embodiment, the one or more plasticizers is/are added after preparing the emulsion or said basic emulsion and before spray-drying. Preferably, said principle liposoluble plasticizer is added after step 2). By adding further non-aqueous constituents to said basic emulsion, the final emulsion to be spray-dried is preferably obtained.

According to an embodiment, the process of the invention further comprises the step of adding a co-plasticizer during step 1), after step 1) but before step 2), or after step 2), but preferably before spray-drying. Preferably, said co-plasticizer, water soluble plasticizer or second plasticizer is added after step 2). The expression "during step 1)" preferably also means "as part of step 1)".

The invention does not exclude the possibility that one or more of said at least one plasticizers is/are added during step 2). It is also noted that step 1) could be decomposed in separate steps, including the addition of the constituents in a specific order.

In accordance with embodiments of the invention, it is possible and even preferred to add said co-plasticizer, watersoluble plasticizer and/or second plasticizer *before* adding said principle plasticizer, said liposoluble plasticizer or said first plasticizer, for example before or during preparing said emulsion, preferably said basic emulsion. This applies, of course, to embodiments where at least two plasticizers are used, one being liposoluble and the other preferably water soluble. In embodiments where only one plasticizer is used, the plasticizer may be added as specified elsewhere in this specification, for example, but preferably after step 2), that is, after preparing said emulsion, preferably said basic emulsion. According to an embodiment, said plasticizer, said principle plasticizer and/or said co-plasticizer is a food- or pharma-grade plasticizer. As the microcapsules of the invention are intended for oral consumption and in particular human or animal consumption, all constituents of said microcapsules are preferably suitable for human or animal consumption, for example as additives to food products or in medicaments or nutritional supplements.

According to an embodiment, said liposoluble plasticizer is selected from the group of triacetin, dibutyl phthalate, dibutyl sebacate, diethyl phthalate, dimethyl phthalate, acetyltributyl citrate, acetyltriethyl citrate, diacetylated monoglycerides, dibutyl sebacate, mineral oil, benzyl benzoate, chlorbutanol, glycerin monostearate, lanolin alcohols, cellulose acetate phthalate compatible.

According to an embodiment, said co-plasticizer and/or water soluble plasticizer is selected from the group of polyvinylpyrrolidone (PVP) and polyvinyl alcohol (PVA).

Said liposoluble plasticizer provides at least 2%, more preferably at least 3% by weight and most preferably at least 4% by weight of the total of the non-aqueous constituents of the emulsion before spray-drying, and said co-plasticizer provides at least 1% by weight, preferably at least 2%, more preferably at least 3% by weight and most preferably at least 4% by weight of the total of the non-aqueous constituents of the emulsion before spray-drying. These percentages also refer to the dry matter content of emulsifiers in the capsules of the invention.

According to an embodiment, the emulsion preferably comprises, in percent by weight of the total of all non-aqueous constituents before spray drying, from 1% to 20%, preferably 2% to 15%, and more preferably 3% to 10% of said plasticizer and/or principle plasticizer and, in addition, from 1% to 20%, preferably 2% to 15% and more preferably 3% to 10% of said co-plasticizer. These percentages also refer to the dry matter content of emulsifiers in the capsules of the invention.

In an embodiment, the one or more plasticizers of the invention is not or is other than one or more selected from monosaccharides and disaccharides, such as sucrose and fructose, and polyols such as glycerol, and polyethylene glycol. In other words, if the microcapsules contain any one or more selected from the group of: monosaccharides and disaccharides, such as sucrose and fructose, and polyols such as glycerol, and polyethylene glycol, said microcapsules contain, in addition, one or more liposoluble plasticizers as disclosed in this specification.

In particular polyols may be present in the microcapsules and/or in the process of the invention. However, such polyols are, if present, added in addition to said one or more plasticizers, in particular in addition to any one of said liposoluble plasticizer, said co-plasticizer and said water soluble plasticizer. In accordance with the invention, polyols (see in particular the polyalcohols mentioned below) are not considered as plasticizers, but may be used or added due to their impact on the organoleptic properties and also due to their synergistic effect if used in combination with the plasticizers and/or with the emulsifiers.

According to an embodiment, the process of the invention comprises the step of adding one or more polyhydric alcohol and/or polyalcohol. For the purpose of this specification, the expressions "polyhydric alcohol" and/or "polyalcohol" are used interchangeably. The polyhydric alcohol may be selected, according to an embodiment, from the group consisting of arrabitol, ribitol, xylitol, galactitol, fucitol, iditol, inositol, volemitol, isomalt, lactitol, maltotriitol, maltotetraitol, polyglicitol, sorbitol, maltitol, mannitol, propylene glycol, polyethyle glycol, dextrin, glycerine, polyvinyl alcohol. According to a preferred embodiment, said polyhydric alcohol is selected from the group consisting of arrabitol, ribitol, xylitol, galactitol, fucitol, iditol, inositol, volemitol, isomalt, lactitol, maltotriitol, maltotetraitol, polyglicitol, sorbitol, maltitol, mannitol. Most preferably, the polyhydric alcohol is selected from the group consisting of xylitol, sorbitol, maltitol, and mannitol. Most preferably, said polyhydric alcohol is xylitol.

The inventors found a synergistic effect of the polyalcohol on the effects of the plasticizers and/or the emulsifiers present in the emulsion. Without wishing to be bound by theory, the inventors speculate that the polyalcohol plays a role of an additional "co-surfactant" and/or as an additional "co-plasticizer". In other words, the polyalcohols appear to have a synergistic effect with respect to the plastification of the polymers and the stability of the emulsion, thereby contributing to the overall beneficial effects of the capsules of the invention.

Said one or more polyhydric alcohol is preferably added in order to improve the organoleptic properties of the capsules, as it preferably adds a slightly sweet taste and is suitable to cover residual off-taste that may originate from the hydrophobic material (marine oil). Furthermore, the polyhydric alcohol may have emulsifying properties and may thus contribute to the stability of the emulsion prepared in the process of the invention, thereby synergistically supporting the emulsifier and/or emulsifier system that is preferably added as disclosed elsewhere in this specification.

According to an embodiment of the process and the capsules of the invention, respectively, the one or more polyhydric alcohol provides at least 0.5% by weight, determined, with respect of the weight of non-aqueous constituents of the emulsion containing all constituents, and, with respect to the dry matter of said microcapsule.

According to an embodiment of the process of the invention, the polyhydric alcohol provides 0.5-20%, preferably 1-10%, more preferably 2-8% and most preferably 2-8% by weight, determined with respect to the weight of the total of the non-aqueous constituents of the emulsion before spray-drying.

According to an embodiment of the microcapsules of the invention, the polyhydric alcohol provides 0.5-20%, preferably 1-10%, more preferably 2-8% and most preferably 2-8% by weight, determined with respect to the weight of the total of the non-aqueous constituents of the emulsion before spray-drying.

According to an embodiment of the process of the invention, the emulsion comprises, in percent by weight of all non-aqueous constituents of the emulsion before spray-drying, 20-70% of polymers, 10-50% of hydrophobic material, 0.5-15% of emulsifiers, and 2-25% of plasticizers.

According to a preferred embodiment of the process of the invention, the emulsion comprises, in percent by weight of all non-aqueous constituents of the emulsion before spray-drying, 30-60% of polymers, 15-45% of hydrophobic material, 1-10% of emulsifiers, and 5-20% of plasticizers.

According to a more preferred embodiment of the process of the invention, the emulsion comprises, in percent by weight of all non-aqueous constituents of the emulsion before spray-drying, 35-55% of polymers, 20-40% of hydrophobic material, 2-8% of emulsifiers, and 6-15% of plasticizers.

The above preferred, more preferred and most preferred percentages of the emulsion also apply to the total dry matter content of the capsules of the invention.

According to another embodiment of the process of the invention, the emulsion comprises, in percent by weight of all non-aqueous constituents of the emulsion before spray-drying, 20-70% of polymers, 10-50% of hydrophobic material, 0.5-15% of emulsifiers, 0.5-15% of polyalcohol, and 2-25% of plasticizers.

According to a preferred embodiment of the process of the invention, the emulsion comprises, in percent by weight of all non-aqueous constituents of the emulsion before spray-drying, 30-60% of polymers, 15-45% of hydrophobic material, 1-10% of emulsifiers, 1-10% of polyalcohol, and 5-20% of plasticizers.

According to a more preferred embodiment of the process of the invention, the emulsion comprises, in percent by weight of all non-aqueous constituents of the emulsion before spray-drying, 35-55% of polymers, 20-40% of hydrophobic material, 2-8% of emulsifiers, 2-8% of polyalcohol, and 6-15% of plasticizers.

In an embodiment, the microcapsules of the invention are free of added protein, or contain, in percent by weight of all non-aqueous constituents, less than 20%, preferably less than 15% more preferably less than 10% and most preferably less than 5% of protein, in particular of film-forming protein.

In an embodiment, the microcapsules of the invention are free of a protective coating, in particular free of a hydrophilic coating, which may be applied by pan coating, fluid bed coating or a coating applied in a rotating enrober drum. This does not exclude the presence of a protective coating in other embodiments of the invention.

The above preferred, more preferred and most preferred percentages of the emulsion also apply to the total dry matter content of the capsules of the invention.

In a preferred embodiment, the percentages apply to the microcapsules after spray-drying and which preferably do not contain any additional coating. In another embodiment, the percentages refer to microcapsules that may or may not contain a further coating, such as a protective coating.

The invention provides edible compositions and/or ingestible compositions and/or products comprising the capsules of the invention. The ingestible composition may be a food product, a food supplement, a pharmaceutical supplement, an animal feed, for example, comprising the microcapsules of the invention. The capsules of the invention may be used as an additive that is added in the course of the manufacturing product of the ingestible composition.

The present invention will now be illustrated by way of examples. These examples do not limit the scope of this invention, which is defined by the appended claims.

### Examples:

The examples below were performed on a lab-scale (Examples 1-10) and pilot plant-scale (Examples 11-12). Examples 13-16 are stability testings performed on the spray-dried powders obtained in Examples 1-4. The following components were used:
Gum arabic: Eficacia M (Nexira et Colloidal Naturel International, Rouen, France).

HPMC: Pharmacoat (Grade 603, Shin-Estsu, Japan).

Surfactants: The surfactant mix used in all examples contained a mix of the Sucrose esters Sisterna® PS750 and Sisterna® SP50 (Sigma Aldrich, Germany) at weight percentages of 85% and 15%, respectively, making up 100% of the surfactants.

Marine Oils: Krill Oil (Aker Supra Krill oil, Aker BioMarine, Norway), Maxomega 46/38 Ethyl ester (BASF Pharma, Scotland), Algatrium DHA70 (Brudy Technology, Spain), Epax 6000 TG/N (Epax, Norway).

Others components used: Xylitol (XyliSorb 90, Roquette, France), Sorbitol (Neosorb P 60W, Roquette, France), Maltitol (SweetPearl P 200, Roquette, France), Mannitol (Pearlitol 25 C, Roquette, France).

Plasticizers: polyvinyl alcohol (PVA, Mowiol 5-88, Kuraray, USA), Triacetin (Sigma, T5376-1L), polyvinylpyrrolidone (PVP, PVP40, Sigma Aldrich).

Emulsification of the lab-scale and pilot plant-scale emulsions were performed using a ultrasound emulsifier (MPI-Ultrasonics, Le Locle, Switzerland) and an APV Homogeniser AS (Type 8.3011, Rannie AS, Denmark).

Spray-drying of the lab-scale emulsions were performed using a B-290 mini spray-dryer (BUCHI Labortechnik AG in Flawil, Switzerland). The inlet temperature was adjusted to 180°C, the outlet temperature 80°C, nozzle cooling was activated. Aspiration was adjusted to 90% and the pump to 20%.

Spray-drying of the pilot plant-scale emulsions were performed using a MOBILE MINOR spray drying apparatus (GEA Process Engineering Inc. 9165 Rumsey Road, Columbia, USA) with the following process parameters: inlet temperature: 140°C, outlet temperature: 85°C, atomization air pressure: 0.65 bar, atomization air flow rate: 7 kg/h, feed flow: 30 g/min.

The emulsions were analyzed by Dynamic Light Scaterring (DLS) in accordance with the international standard ISO 13320-1 using the NICOMP™ 380 ZLS analyzer.

### Example 1: Encapsulating krill oil (30 weight-%) by spray-drying

The following ingredients were provided at the indicated weight percentages, so as to make up for 50 g of non-aqueous constituents: 18% HPMC (9 g), 18% gum arabic, 30% krill oil, 10% of surfactants mix, 10% triacetin (liposoluble plasticizer), 10% PVP (watersoluble plasticizer), and 4 % xylitol.

The HPMC, gum arabic, xylitol and the surfactants mix were solubilized in 200 mL demineralized water. Once solubilized, the solution is let cool down to room temperature (25°C) to yield solution 1.

Krill oil was supplemented with a tocopherol mix, labiatae-plant alcohol extract (35%) and metal chelater for avoiding oxidation. The surfactants were solubilized to yield solution 2. Solution 2 was added to solution 1 and emulsified therein in three cycles of two minutes each at 85% amplitude.

Polyvinylpyrrolidone (PVP) was dissolved in 200 mL of demineralized water, let cool down to room temperature and added to the emulsion. Thereafter, triacetin was added and all components were well mixed.

Analysis by DLS yielded emulsion droplet diameter of 301.9 nm with a standard deviation of 0.125 nm and a variance (PI) value of 0.035.

Thereafter, the emulsion was spray-dried. Immediately after collecting the spray-dried particles, it was surprisingly noted that the typical odor of marine oil was absent or at least reduced to a unexpected extent.

### Example 2: Encapsulating krill oil (25 weight-%) by spray-drying

The following ingredients were provided at the indicated weight percentages, so as to make up for 50 g of non-aqueous constituents: 22% HPMC, 22% gum arabic, 25% krill oil, 10% of surfactants mix, 8% triacetin (liposoluble plasticizer), 8% PVA (watersoluble plasticizer), and 5 % xylitol.

The HPMC, gum arabic, xylitol and the mix surfactants were solubilized in 200 mL demineralized water. Once solubilized, the solution was let cool down to room temperature (25°C) to yield solution 1.

Krill oil was supplemented with antioxidants, plant extract and metal chelator as described in Example 1 and solubilized to yield solution 2. Solution 2 was added to solution 1 and emulsified therein as described in Example 1.

Thereafter triacetin was added to the emulsion. Polyvinyl alcohol (PVA) is dissolved in 200 mL of demineralized water and also added to the emulsion. All components were well mixed.

Analysis by DLS yielded emulsion droplet diameter of 291.2 nm with a standard deviation of 0.145 nm and a variance (PI) value of 0.017.

Thereafter, the emulsion was spray-dried.

### Example 3: Encapsulating fish oil (30 weight-%) by spray-drying (not according to the invention)

The following ingredients were provided at the indicated weight percentages, so as to make up for 50 g of non-aqueous constituents: 25% HPMC, 25% gum arabic, 30% Maxomega fish oil, 5% of mix surfactants, and 10% PVP (watersoluble plasticizer), and 5 % xylitol. HPMC and gum arabic were added to 350 mL of distilled water and solubilized under heating. 2.5 g of Xylitol was added to the solution and mixed. Surfactants were added separately to 100 mL of distilled water and solubilized under heating. The surfactant solution was added to the solution with the matrix materials, all components were well mixed. The solution was then let cool down to room temperature. The fish oil was added to the solution and the entire solution was emulsified as described in Example 1.

Separately, PVP was solubilized in 50 mL of distilled water and added to the emulsion.

Analysis of the particles by DLS yielded emulsion droplet diameter of 255.2 nm with a standard deviation of 0.130 nm and a variance (PI) value of 0.042.

Following mixing, the emulsion containing all components was spray-dried.

### Example 4: Encapsulating fish oil (30 weight-%) by spray-drying

The following ingredients were provided at the indicated weight percentages, so as to make up for 50 g of non-aqueous constituents: 25% HPMC, 25% gum arabic, 30% Maxomega fish oil, 5% of mix surfactants, 5% PVP, 5% triacetin and 5 % xylitol.

HPMC and gum arabic were added to 350 mL of distilled water and solubilized under heating. 2.5 g of Xylitol was added to the solution and mixed. Surfactants were added separately to 100 mL of distilled water and solubilized under heating. The surfactant solution was added to the solution with the matrix materials, all components were well mixed. The solution was then let cool down to room temperature. The fish oil was added to the solution and the entire solution was emulsified as described in Example 1.

Separately, PVP was solubilized in 50 mL of distilled water and added to the emulsion. Thereafter, triacetin was also added to the emulsion and all components were well mixed. Analysis of the particles by DLS yielded emulsion droplet diameter of 274.3 nm with a standard deviation of 0.147 nm and a variance (PI) value of 0.031.

Following mixing, the emulsion containing all components was spray-dried.

### Example 5: Encapsulating Epax fish oil (30 weight-%) by spray-drying (not according to the invention)

The following ingredients were provided at the indicated weight percentages, so as to make up for 50 g of non-aqueous constituents: 25% HPMC, 25% gum arabic, 30% Epax fish oil, 5% xylitol, 5% of mix surfactants and 10% PVA.

HPMC and gum arabic were added to 350 mL of distilled water and solubilized under heating. 2.5 g of xylitol was added to the solution and mixed. Surfactants were added to 100 mL of distilled water and solubilized under heating. The surfactant solution was added to the solution with the matrix materials, all components were well. The solution was then let cool down. The fish oil was added to the solution and the entire solution was emulsified as described in Example 1.

Separately, the PVA was solubilized in 50 mL of distilled water and added to the emulsion. All components were well mixed. Analysis of the particles by DLS yielded emulsion droplet diameter of 248.7 nm with a standard deviation of 0.109 nm and a variance (PI) value of 0.023.

Following mixing, the emulsion containing all components was spray-dried.

### Example 6: Encapsulating Maxomega fish oil (30 weight-%) by spray-drying

The following ingredients were provided at the indicated weight percentages, so as to make up for 50 g of non-aqueous constituents: 25% HPMC, 25% gum arabic, 30% Maxomega fish oil, 5% xylitol, 5% of surfactant mix, 5% PVA and 5% triacetin.

HPMC and gum arabic were added to 350 mL of distilled water and solubilized under heating. 2.5 g of xylitol was added to the solution and mixed. Surfactants were added to 100 mL of distilled water and solubilized under heating. The surfactant solution was added to the solution with the matrix materials, all components were well mixed. The solution was then let cool down. The fish oil was added to the solution and the entire solution was emulsified as described in Example 1.

Separately, the PVA was solubilized in 50 mL of distilled water and added to the emulsion. Triacetin is also added to the emulsion. All components were well mixed. Analysis of the particles by DLS yielded emulsion droplet diameter of 250.1 nm with a standard deviation of 0.129 nm and a variance (PI) value of 0.040.

Following mixing, the emulsion containing all components was spray-dried.

### Example 7: Encapsulating Epax fish oil (30 weight-%) by spray-drying

The following ingredients were provided at the indicated weight percentages, so as to make up for 50 g of non-aqueous constituents: 25% HPMC, 25% gum arabic, 30% EPAX fish oil, 5% xylitol, 5% of surfactant mix, and 10% triacetin.

HPMC and gum arabic were added to 400 mL of distilled water and solubilized under heating. 2.5 g of xylitol was added to the solution and mixed. Surfactants were added to 100 mL of distilled water and solubilized under heating. The surfactant solution was added to the solution with the matrix materials, all components were well mixed. The solution was then let cool down. The fish oil was added to the solution and the entire solution was emulsified as described in Example 1.

Thereafter, triacetin is also added to the emulsion. All components were well mixed. Analysis of the particles by DLS yielded emulsion droplet diameter of 275.9 nm with a standard deviation of 0.121 nm and a variance (PI) value of 0.026.

Following mixing, the emulsion containing all components was spray-dried.

### Example 8: Encapsulating Epax fish oil (40 weight-%) by spray-drying

The following ingredients were provided at the indicated weight percentages, so as to make up for 50 g of non-aqueous constituents: 20% HPMC, 20% gum arabic, 40% EPAX fish oil, 5% xylitol, 5% of surfactant mix, 5% PVA and 5% triacetin.

HPMC and gum arabic were added to 350 mL of distilled water and solubilized under heating. 2.5 g of xylitol was added to the solution and mixed. Surfactants were added to 100 mL of distilled water and solubilized under heating. The surfactant solution was added to the solution with the matrix materials, all components were well mixed. The solution was then let cool down. The fish oil was added to the solution and the entire solution was emulsified as described in Example 1.

Separately, the PVA was solubilized in 50 mL of distilled water and added to the emulsion. Triacetin is also added to the emulsion. All components were well mixed. Analysis of the particles by DLS yielded emulsion droplet diameter of 288.1 nm with a standard deviation of 0.113 nm and a variance (PI) value of 0.021.

Following mixing, the emulsion containing all components was spray-dried.

### Example 9: Encapsulating Algatrium (30 weight-%) by spray-drying

The following ingredients were provided at the indicated weight percentages, so as to make up for 50 g of non-aqueous constituents: 25% HPMC, 25% gum arabic, 30% Algatrium fish oil, 5% xylitol, 5% of surfactant mix, 5% PVA and 5% triacetin.

HPMC and gum arabic were added to 350 mL of distilled water and solubilized under heating. 2.5 g of xylitol was added to the solution and mixed. Surfactants were added to 100 mL of distilled water and solubilized under heating. The surfactant solution was added to the solution with the matrix materials, all components were well. The solution was then let cool down. The fish oil was added to the solution and the entire solution was emulsified as described in Example 1.

Separately, the PVA was solubilized in 50 mL of distilled water and added to the emulsion. Triacetin is also added to the emulsion. All components were well mixed. Analysis of the particles by DLS yielded emulsion droplet diameter of 285.2 nm with a standard deviation of 0.188 nm and a variance (PI) value of 0.030.

Following mixing, the emulsion containing all components was spray-dried.

### Example 10: Encapsulating Algatrium (30 weight-%) by spray-drying (triacetin only)

The following ingredients were provided at the indicated weight percentages, so as to make up for 50 g of non-aqueous constituents: 25% HPMC, 25% gum arabic, 30% Algatrium fish oil, 5% xylitol, 5% of surfactant mix, and 10% triacetin.

HPMC and gum arabic were added to 400 mL of distilled water and solubilized under heating. 2.5 g of xylitol was added to the solution and mixed. Surfactants were added to 100 mL of distilled water and solubilized under heating. The surfactant solution was added to the solution with the matrix materials, all components were well. The solution was then let cool down. The fish oil was added to the solution and the entire solution was emulsified as described in Example 1.

Thereafter, triacetin is added to the emulsion. All components were well mixed. Analysis of the particles by DLS yielded emulsion droplet diameter of 271.9 nm with a standard deviation of 0.145 nm and a variance (PI) value of 0.047.

Following mixing, the emulsion containing all components was spray-dried.

### Example 11: Scale-up encapsulation of fish oil by spray-drying with cooling nozzle

The following ingredients were provided at the indicated weight percentages, so as to make up for 1 kg of non-aqueous constituents: 25% HPMC, 25% gum arabic, 30% EPAX fish oil, 5% xylitol, 5% of surfactant mix, 5% PVA and 5% triacetin.

HPMC and gum arabic were added to 4000 mL of distilled water and solubilized under heating. Xylitol was added to the solution and mixed. Surfactants were added to 300 mL of distilled water and solubilized under heating. The surfactant solution was added to the solution with the matrix materials, all components were well. The solution was then let cool down. The fish oil was added to the solution and the entire solution was emulsified as described in Example 1.

Separately, PVA is solubilized in 200 mL of distilled water and added to the emulsion. Thereafter, triacetin is added to the emulsion. All components were well mixed. Analysis of the particles by DLS yielded emulsion droplet diameter of 271.9 nm with a standard deviation of 0.145 nm and a variance (PI) value of 0.047.

Following mixing, the emulsion containing all components was spray-dried (for process parameters, see above "Examples").

Furthermore, the dry particles were analyzed by DLS in accordance with ISO 13320-1 using the NICOMP TM 380 ZLS analyzer. The mean particles size (diameter) was found to be 5.34 µm with a standard deviation of 0.453 µm and variance (PI) value of 0.056.

### Example 12: Scale-up encapsulation of fish oil by spray-drying with cooling nozzle

Example 11 is repeated with the only difference being that the nozzle was not cooled during spray-drying.

### Example 13: Stability studies

The relative humidity (water content), peroxide index and the Onset Oxidation Temperature (OOT) of the microcapsules obtained in Examples 1 to 12 were analyzed following spray-drying. The analysis was repeated for the microcapsules of Examples 1 to 4 following 3 and 6 months of storage in semi permeable bags at a relative humidity of 75%, a temperature of 40°C. The results obtained directly after spray-drying are shown in Table 1 below:

**Table 1: Stability parameters of microcapsules following spray-drying**

| Example | Time = 0 month | | |
|---|---|---|---|
| | Relative humidity(%) | Peroxide Indice(meq/Kg oil) | OOT (°C) |
| 1 | 2.056 | 0.12 | 178 |
| 2 | 1.963 | 0.20 | 179 |
| 3 | 2.300 | 0.25 | 160 |
| 4 | 1.892 | 0.32 | 159 |
| 5 | 2.350 | 0.186 | 166 |
| 6 | 2.100 | 0.192 | 169 |
| 7 | 2.020 | 0.175 | 165 |
| 8 | 1.980 | 0.125 | 168 |
| 9 | 2.310 | 0.32 | 159 |
| 10 | 2.180 | 0.29 | 158 |
| 11 | 1.008 | 0.2 | 170 |
| 12 | 0.950 | 0.23 | 169 |
| Krill oil* | 1.52 | 0.09 | 164 |
| Maxomega* | 0.55 | 0.17 | 162 |

The results obtained after storage are shown in Table 2 below:

**Table 2: Stability parameters of spray-dried microcapsules after 3 and 6 months of storage**

| Example | Time = 3 months | | | Time = 6 months | | |
|---|---|---|---|---|---|---|
| | Relative humidity (%) | Peroxide Indice (meq/Kg oil) | OOT (°C) | Relative humidity (%) | Peroxide Indice (meq/Kg oil) | OOT (°C) |
| 1 | 2.056 | 0.18 | 178 | 2.056 | 0.23 | 178 |
| 2 | 1.963 | 0.31 | 179 | 1.963 | 0.36 | 179 |
| 3 | 2.309 | 0.29 | 160 | 2.317 | 0.31 | 160 |
| 4 | 1.895 | 0.34 | 159 | 1.899 | 0.37 | 159 |
| Krill oil* | 1.57 | 0.17 | 160 | 1.64 | 0.39 | 150 |
| Maxomega* | 0.58 | 0.27 | 159 | 0.85 | 0.45 | 147 |

| | | | | | | |
|---|---|---|---|---|---|---|
| * Non-encapsulated. | | | | | | |

From Tables 1 and 2 it is apparent that the measured oxydation indicators (the Peroxide Index and the Oxydation Onset Temperature, OOT), are much better for all the products of microencapsulation as represented by the trials 1 to 12 after 3 month storage than the non encapsulated krill oil and Maxomega oil. These values are significantly different after 6 month. This demonstrates the efficiency of the microencapsulation in accordance with the current invention.

## Claims

1. A process for microencapsulating a hydrophobic material comprising PUFAs, the process comprising the steps of:
- preparing a micro emulsion comprising water, one or more polymers, said hydrophobic material, and at least one emulsifier, wherein said hydrophobic material forms droplets having an average size of 1 µm or smaller in said micro emulsion,
- adding at least one liposoluble plasticizer, said liposoluble plasticizer providing at least 2% of the weight of said micro-emulsion, determined with respect to the weight of the non-aqueous constituents of the micro-emulsion containing all constituents and/or immediately before spray drying; and,
- spray-drying said micro-emulsion.

2. The process of claim 1, wherein said liposoluble plasticizer is added after preparing said emulsion and/or is added to the emulsion before spray-drying.

3. The process of claim 1 or 2, wherein the process further comprises the step of adding a co-plasticizer so as to produce an emulsion comprising at least two plasticizers.

4. The process of claim 3, wherein said step of preparing said emulsion comprises the steps of:
1) mixing said one or more polymers, water, said hydrophobic material and said at least one emulsifier to produce a mixture; and,
2) emulsifying said mixture so as to obtain said emulsion;
and wherein said co-plasticizer is added during step 1), after step 1) but before step 2), or after step 2).

5. The process of any one of the preceding claims, wherein said liposoluble plasticizer is selected from the group of triacetin, dibutyl phthalate, dibutyl sebacate, diethyl phthalate, dimethyl phthalate, acetyltributyl citrate, acetyltriethyl citrate, diacetylated monoglycerides, dibutyl sebacate, mineral oil, benzyl benzoate, chlorbutanol, glycerin monostearate, lanolin alcohols, cellulose acetate phthalate compatible.

6. The process of any one of claims 3-5, wherein said co-plasticizer is a water soluble plasticizer.

7. The process of claim 6, wherein said water soluble plasticizer is selected from the group of polyvinylpyrrolidone (PVP) and polyvinyl alcohol (PVA).

8. The process of any one of the preceding claims, wherein said at least one liposoluble plasticizer and, if present, said co-plasticizer, is/are added so as to provide 3% to 35% by weight of all non-aqueous constituents of the micro-emulsion before spray drying.

9. The process of any one of the preceding claims, further comprising the step of adding a polyhydric alcohol selected from the group consisting of arrabitol, ribitol, xylitol, galactitol, fucitol, iditol, inositol, volemitol, isomalt, lactitol, maltotriitol, maltotetraitol, polyglicitol, sorbitol, maltitol, mannitol, propylene glycol, polyethyle glycol, dextrin, glycerine, polyvinyl alcohol.

10. The process of any one of the preceding claims, wherein said hydrophobic material comprises a marine oil, preferably krill oil and/or fish oil and/or modified marine oil.

11. The process of any one of the preceding claims, wherein the emulsion comprises, in percent by weight of the total of the non-aqueous constituents of the emulsion before spray-drying, 5% to 50% by weight of said hydrophobic material.

12. The process of any one of the preceding claims, wherein said one or more polymers comprise one or both selected from cellulose derivatives and pullulan, and, in addition, a natural gum.

13. The process of claim 12, wherein said cellulose derivatives are preferably selected from alkylated, hydroxyalkylated and/or carboxyalkylated cellulose.

14. The process of any one of claims 12-13, wherein said emulsion comprises, in percent by weight of the total of the non-aqueous constituents of the emulsion before spray-drying, at least 10% by weight of dry matter of said cellulose derivate and/or pullulan, and, in addition, at least 10% by weight of dry matter of said natural gum.

15. Microcapsules obtainable by the process according to any one of claims 1 to 14.

16. A microcapsule comprising a matrix comprising polymers and droplets of a hydrophobic material dispersed in said matrix, said droplets having an average diameter of 1 µm or smaller, said hydrophobic material comprising PUFAs, wherein said hydrophobic material provides at least 10% per weight of dry matter of said microcapsule, **characterized in that** said microcapsule comprises a lipophilic plasticizer, which provides at least 2% per weight of dry matter of said microcapsule.

17. An ingestible product comprising the microcapsules of claim 15 and/or claim 16.

## Patentansprüche

1. Verfahren zur Mikroverkapselung eines hydrophoben Materials, das PUFAs umfasst, wobei das Verfahren die folgenden Schritte umfasst:
- Herstellung einer Mikroemulsion, die Wasser, ein oder mehrere Polymere, das besagte hydrophobe Material und mindestens einen Emulgator umfasst, wobei das besagte hydrophobe Material Tröpfchen mit einer durchschnittlichen Größe von 1 µm oder weniger in der besagter Mikroemulsion hatte,
- Hinzufügen von mindestens einem fettlöslichen Weichmachers, wobei der besagter fettlösliche Weichmacher mindestens 3 Gew .-% der Mikroemulsion bereitstellt, bestimmt in Bezug auf das Gewicht der nichtwäßrigen Bestandteile der Mikroemulsion, die alle Bestandteile enthält, und/oder unmittelbar vor der Sprühtrocknung; und,
- Sprühtrocknen der Mikroemulsion.

2. Verfahren nach Anspruch 1, wobei der besagter fettlösliche Weichmacher nach der Herstellung der Emulsion zugegeben wird und/oder der Emulsion vor dem Sprühtrocknen zugesetzt wird.

3. Verfahren nach Anspruch 1 oder 2, wobei das Verfahren ferner den Schritt des Zugebens eines Co-Weichmachers umfasst, um eine Emulsion herzustellen, die mindestens zwei Weichmacher umfasst.

4. Verfahren nach Anspruch 3, wobei der besagter Schritt des Herstellens der besagte Emulsion die folgenden Schritte umfasst:
1. Mischen des besagten einen oder der mehreren Polymere, Wasser, des besagten hydrophoben Materials und des besagten mindestens einen Emulgators, um eine Mischung herzustellen; und,
2. Emulgieren der besagten Mischung, um die besagte Emulsion zu erhalten;
und der besagte Co-Weichmacher zugegeben wird während Schritt 1), nach Schritt 1), aber vor Schritt 2) oder nach Schritt 2).

5. Verfahren nach einem der vorhergehenden Ansprüche, wobei der fettlösliche Weichmacher ausgewählt ist aus der Gruppe von Triacetin, Dibutylphthalat, Dibutylsebacat , Diethylphthalat, Dimethylphthalat, Acetyltributylcitrat, Acetyltriethylcitrat, diacetylierte Monoglyceride, Dibutylsebacat, Mineralöl, Benzylbenzoat, Chlorbutanol, Glycerinmonostearat, Lanolinalkohole, Celluloseacetatphtalat-kompatibel.

6. Verfahren nach einem der Ansprüche 3 bis 5, wobei der Co-Weichmacher ein wasserlöslicher Weichmacher ist.

7. Verfahren nach Anspruch 7, wobei der wasserlösliche Weichmacher aus der Gruppe Polyvinylpyrrolidon (PVP) und Polyvinylalkohol (PVA) ausgewählt ist.

8. Verfahren nach einem der vorangehenden Ansprüche, wobei der besagte mindestens eine fettlösliche Weichmacher und, falls vorhanden, der besagter Co-Weichmacher zugegeben wird/werden, um vor dem Sprühtrocknen 4 bis 35 Gew .-% aller nichtwäßrigen Bestandteile der Mikroemulsion dazustellen.

9. Verfahren nach einem der vorhergehenden Ansprüche, ferner umfassend den Schritt des Zugebens eines mehrwertigen Alkohols, ausgewählt aus der Gruppe bestehend aus Arabit, Ribit, Xylit, Galactit, Fucit, Iditol, Inosit, Volemitol, Isomalt, Lactitol, Maltotriitol, Maltotetraitol, Polyglicitol, Sorbit, Maltit, Mannit, Propylenglykol, Polyethylglukol, Glykol, Dextrin, Glycerin, Polyvinylalkohol.

10. Verfahren nach einem der vorhergehenden Ansprüche, wobei das hydrophobe Material ein Meeresöl, vorzugsweise Krillöl und/oder Fischöl und/oder ein modifiziertes Meeresöl umfasst.

11. Verfahren nach einem der vorhergehenden Ansprüche, wobei die Emulsion in Gewichtsprozent der Gesamtmenge der nichtwäßrigen Bestandteile der Emulsion vor dem Sprühtrocknen 5 bis 50 Gew .-% des hydrophoben Materials umfasst.

12. Verfahren nach einem der vorhergehenden Ansprüche, wobei das eine oder die mehreren Polymere eines oder beide ausgewählt aus Cellulosederivaten und Pullulan und zusätzlich einen Naturgummi umfassen.

13. Verfahren nach Anspruch 12, wobei die Cellulosederivate vorzugsweise ausgewählt sind aus alkylierter, hydroxylalkylierter und/oder carboxyalkilierter Cellulose.

14. Verfahren nach einem der Ansprüche 12-13, wobei die besagte Emulsion umfasst in Gew .-% der Gesamtmenge der nichtwäßrigen Bestandteile der Emulsion vor dem Sprühtrocknen mindestens 10 Gew .-% der Trockensubstanz des Cellulose Derivates und/oder Pullulan und zusätzlich mindestens 10 Gew .-% Trockensubstanz des natürlichen Gummis.

15. Mikrokapseln, erhältlich durch das Verfahren nach einem der Ansprüche 1 bis 14.

16. Eine Mikrokapsel, umfassend eine Matrix, die Polymere und Tröpfchen eines in der Matrix dispergierten hydrophoben Materials umfasst, wobei die Tröpfchen einen durchschnittlichen Durchmesser von 1 m oder weniger aufweisen, wobei das hydrophobe Material PUFAs umfasst, wobei das hydrophobe Material mindestens 10 Gew .-% der Trockensubstanz der Mikrokapsel bereitstellt, **dadurch gekennzeichnet, dass** die Mikrokapsel einen lipophilen Weichmacher umfasst, der mindestens 2 Gew .-% Trockensubstanz der Mikrokapsel bereitstellt.

17. Ein einnehmbares Produkt, umfassend die Mikrokapseln nach Anspruch 15 und/oder Anspruch 16.

## Revendications

1. Procédé de micro encapsulation d'un matériau hydrophobe comprenant des PUFA, le procédé comprenant les étapes consistant à:
- préparation d'une microémulsion comprenant de l'eau, un ou plusieurs polymères, ledit matériau hydrophobe et au moins un émulsifiant, dans lequel ledit matériau hydrophobe forme des gouttelettes ayant une taille moyenne de 1 µm ou moins dans ladite microémulsion,
- addition d'au moins un plastifiant liposoluble, ledit plastifiant liposoluble fournissant au moins 2% du poids de la microémulsion, déterminé par rapport au poids des constituants non aqueux de la microémulsion contenant tous les composants et/ou immédiatement avant le séchage par pulvérisation; et,
- séchage par pulvérisation ladite microémulsion.

2. Procédé selon la revendication 1, dans lequel ledit plastifiant liposoluble est ajouté après la préparation de ladite émulsion et/ou est ajouté à l'émulsion avant le séchage par pulvérisation.

3. Procédé selon la revendication 1 ou 2, dans lequel le procédé comprend en outre l'étape consistant à ajouter un co-plastifiant de manière à produire une émulsion comprenant au moins deux plastifiants.

4. Procédé selon la revendication, dans lequel ladite étape de préparation de ladite émulsion comprend les étapes consistant à:
1. Mélanger ledit ou lesdits polymères, de l'eau, ledit matériau hydrophobe et ledit au moins un émulsifiant pour produire un mélange, et,
2. émulsionner ledit mélange afin d'obtenir ladite émulsion;
et ledit co-plastifiant est ajouté pendant l'étape 1), après l'étape 1), mais avant l'étape 2) ou après l'étape 2).

5. Procédé selon l'une quelconque des revendications précédentes, dans lequel ledit plastifiant liposoluble est choisi dans le groupe suivant: tri acétine, le phtalate de di butyle, le sébaçate de di butyle, le phtalate de diéthyle, le phtalate de di méthyle, le citrate d'acétyle tributyle, le citrate de diacétyl tri étyle, le mono glycérides di-acétylés, sébacate de di butyle, huile minérale, benzoate de benzyle, chlorobutanol, mono stéarate de glycérol, alcools de lanoline, acétophtalate de cellulose compatible.

6. Procédé selon l'une quelconque des revendications 3 à 5, dans lequel ledit co-plastifiant est un plastifiant soluble dans l'eau.

7. Procédé selon la revendication 7, dans lequel ledit plastifiant soluble dans l'eau est choisi dans le groupe constitué de polyvinylpyrrolidone (PVP) et d'alcool polyvinylique (PVA).

8. Procédé selon l'une quelconque des revendications précédentes, dans lequel on ajoute ledit au moins un plastifiant liposoluble et, s'il est présent, ledit co-plastifiant, de manière à fournir 3% à 35% en poids de tous les constituants non aqueux de la microémulsion avant séchage par pulvérisation.

9. Procédé selon l'une quelconque des revendications précédentes, comprenant en outre l'étape consistant à ajouter un alcool polyhydrique choisi dans le groupe comprenant l'arabitol, le ribitol, le xylitol, le galactitol, le fucitol, iditol, inositol, volemitol, isomalt, lactitol, maltotriitol, maltotétraitol, polyglicitol, sorbitol, maltitol, mannitol, propylène glycol, polyéthyle glucol, glycol, dextrine, glycérine, alcool polyvinylique.

10. Procédé selon l'une quelconque des revendications précédentes, dans lequel ledit matériau hydrophobe comprend une huile marine, de préférence une huile de krill et / ou une huile de poisson et / ou une huile marine modifiée.

11. Procédé selon l'une quelconque des revendications précédentes, dans lequel l'émulsion comprend, en pourcentage en poids du total des constituants non aqueux de l'émulsion avant séchage par pulvérisation, 5% à 50% en poids dudit matériau hydrophobe.

12. Procédé selon l'une quelconque des revendications précédentes, dans lequel ledit ou lesdits polymères comprennent un ou les deux choisis parmi les dérivés de la cellulose et le pullulane, et en outre une gomme naturelle.

13. Procédé selon la revendication 12, dans lequel lesdits dérivés de la cellulose sont de préférence choisis parmi la cellulose alkylée, hydroxy alkylée et/ou carboxy alkylée.

14. Procédé selon l'une quelconque des revendications 12 à 13, dans lequel ladite émulsion comprend, en pourcentage en poids du total des constituants non aqueux de l'émulsion avant séchage par pulvérisation, au moins 10% en poids de la matière sèche de ladite dérivé de cellulose et/ou pullulane et, en outre, au moins 10% en poids de la matière sèche de ladite gomme naturelle.

15. Microcapsules pouvant être obtenues par le procédé selon l'une quelconque des revendications 1 à 14.

16. Une microcapsule comprenant une matrice comprenant des polymères et des gouttelettes d'un matériau hydrophobe dispersé dans ladite matrice, lesdites gouttelettes ayant un diamètre moyen de 1 µm ou moins, ledit matériau hydrophobe comprenant des PUFA, ledit matériau hydrophobe fournissant au moins 10% en poids de matière sèche de ladite microcapsule, **caractérisé en ce que** ladite microcapsule comprend un plastifiant lipophile, qui fournit au moins 2% en poids de matière sèche de ladite microcapsule.

17. Un produit ingérable comprenant les microcapsules de la revendication 15 et/ou de la revendication 16
